# EUROPEAN PATENT SPECIFICATION

(11) **EP 1 699 399 B1**
(45) Date of publication and mention of the grant of the patent: **18.04.2007**
(21) Application number: 04800316.4
(22) Date of filing: 15.11.2004
(51) Int. Cl.: A61F 13/15

(54) **ABSORBENT ARTICLE AND METHOD FOR MANUFACTURING SUCH ARTICLE**
SAUGFÄHIGER ARTIKEL UND VERFAHREN ZUR HERSTELLUNG DIESES ARTIKELS
ARTICLE ABSORBANT ET PROCEDE DE FABRICATION DUDIT ARTICLE

(30) Priority: 29.12.2003 SE 0303556
(43) Date of publication of application: 13.09.2006
(73) Proprietor: SCA Hygiene Products AB, 405 03 Göteborg (SE)
(72) Inventor: KLING, Robert, S-511 63 Skene (SE); DOVERBO, Anna-Gerd, S-431 43 Mölndal (SE)
(74) Representative: Andersson, Per Rune
(86) International application number: PCT/SE2004/001653
(87) International publication number: WO 2005/063159

(56) References cited:
- EP-A2- 0 304 957
- EP-A2- 1 138 301
- US-A- 5 536 555
- US-A- 5 804 021

## Description

### TECHNICAL FIELD

The present invention relates to an absorbent article such as a diaper, pant diaper, an incontinence shield, a sanitary towel or the like, comprising an upper, preferably liquid-permeable cover sheet, a lower, preferably liquid-impermeable cover sheet, and an absorption body arranged between the cover sheets, said article defining a longitudinal direction, a front portion in the longitudinal direction, and a rear portion in the longitudinal direction.

The present invention also relates to a method for manufacturing an absorbent article such as a diaper, an incontinence shield, a sanitary towel or the like, comprising an upper, liquid-permeable cover sheet, a lower, preferably liquid-impermeable cover sheet, and an absorption body arranged between the cover sheets, said article defining a longitudinal direction, a front portion in the longitudinal direction, and a rear portion in the longitudinal direction.

### BACKGROUND ART

In connection with absorbent articles such as diapers, pant diapers, incontinence shields for adults, and sanitary towels, there has long been a general need for materials and structures which are able to take up, distribute and absorb bodily excretions in a rapid and effective manner. Today's absorbent articles generally have good absorbency with a low risk of leakage and a high degree of comfort for the person wearing the absorbent article.

The requirement for effective absorbency in an absorbent article is important not least in the case of diapers for infants where there is a need for rapid uptake, distribution and absorption of urine and excrement. In this connection, it should be noted that newborn babies and also slightly older infants often produce excrement which is loose and runny in consistency. In today's diapers for infants, there is, for example, a risk of this loose excrement leaking out along the back of the person wearing the diaper. Such leakage may entail a risk of, for example, soiled clothes and bed linen. In general, it may be stated that, in connection with diapers for infants, ever greater demands are being placed on the ability to take up excrement via the surface material and absorption material of a diaper.

According to the prior art, a diaper can be designed with so-called extra inner barriers (inner leg gatherers) which form a seal by means of which excrement can be prevented from running out along the sides of the diaper. Another previously known way of achieving this is to use extra transverse pockets in the diaper, these pockets being designed to prevent leakage at the front or rear edge of the diaper.

With the two abovementioned solutions, leakage of excrement from a diaper is prevented in an effective manner. However, the problem still remains that the excrement can stay on the cover sheet of the diaper and soil the user. In addition, there is a risk of the user experiencing skin irritation in such a situation. This problem has in turn been solved in a known way by using a diaper with an extra cover sheet which comprises relatively large openings, for example a type of opening for taking up urine and excrement.

These solutions provide better protection for certain areas of the skin since the extra cover sheet isolates them. However, the problem still remains here that the excrement cannot penetrate down into the diaper. According to the prior art, this problem has been solved by using special cover sheets with small openings which also allow excrement to penetrate down into the structure.

The problem of the abovementioned solutions is that the absorption material which is located under the cover sheet that has been provided with openings can emerge through the openings and attach to the user's skin. This can involve cellulose fibres, superabsorbent particles or loose fibres of polymer material. This is unsatisfactory because it gives a visual impression of a diaper of poor quality. In addition, it makes cleaning the user's skin difficult, and in the worst cases the user may experience skin irritation.

The reason why the previously known cover sheets with openings do not function satisfactorily is that the openings extend in the x-y plane of the diaper (i.e. an imagined plane extending parallel to the surface of the diaper). The underlying absorption material is exposed in this way.

To get round this problem, it is already known to produce a diaper with openings which extend in the z plane of the diaper (i.e. at right angles to the x-y plane). An example of such a solution is described in Swedish patent number SE 449298.

A disadvantage of the solution according to SE 449298, however, is that the openings in the z direction can be easily compressed when the cover sheet is exposed to pressure during use.

There is therefore a need to solve the problem concerning further improved uptake, distribution and absorption of, in particular, loose excrement in the type of diapers which are used on infants and which are based on openings being formed in the cover sheet of a diaper.

### DISCLOSURE OF THE INVENTION

A principal object of the present invention is to make available an improved absorbent article in which the abovementioned problems are solved and the requirements and needs are satisfied.

The above objects are achieved with an absorbent article of the type mentioned in the introduction, which article is characterized in that the upper cover sheet, together with an underlying wadding material, defines a laminate which, at least in said rear portion, is designed with at least one through-slit which defines a corresponding opening for passage of bodily excretions through said slit, and in that said slit is surrounded by binding areas which, together with a resilient action of the wadding material, define a raised area on the surface of said article.

The above objects are also achieved by a method for manufacturing an absorbent article of the type mentioned in the introduction, which method is characterized in that it comprises joining together a laminate which consists of said upper cover sheet in conjunction with an underlying wadding material, forming at least one slit in said laminate over a predetermined surface along said article, by which means a corresponding opening is defined for passage of bodily excretions through said slit, and forming binding areas which surround said slit and which, together with a resilient action of the wadding material, define raised areas on the surface of said article.

The invention affords certain advantages. In particular, it may be noted that the invention permits a high speed of admission of excreted bodily fluid and excrement, which significantly reduces the risk of the liquid or excrement streaming out across the surface of the sheet instead of penetrating through the surface. According to a first embodiment of the invention, in which the abovementioned slits are oriented such that a number of hump-shaped areas are formed, excrement can be transferred in through a top sheet and onwards to a wadding material through the open slits, after which it is spread and is finally bound in the underlying wadding material. In this way, the slits and the hump-shaped areas act to prevent excrement leaking out at the user's back. In addition, excrement accumulates in the valleys between said hump-shaped areas. Since the user's skin lies against the top of the humps, which are substantially free from excrement, there is less skin irritation and a higher degree of user comfort.

According to a second embodiment of the invention, in which the abovementioned slits define substantially straight lines extending substantially transversely across the top face of the article, between the latter's side edges, raised areas are obtained which are defined across the top face of the article. In this way, a structure is obtained which effectively slows down and prevents excrement from leaking out at the user's back.

### DESCRIPTION OF THE FIGURES

The invention will be described below with reference to preferred embodiments and to the attached drawings, in which:
- Figure 1: shows a perspective view of an absorbent article in the form of a diaper for infants, in which diaper the present invention can be used,
- Figure 2: is a cross-sectional view of the diaper according to Figure 1, along the line I-I,
- Figure 3: is a slightly enlarged perspective view of the diaper according to Figures 1 and 2, showing the main features of the invention in detail,
- Figure 4: is a perspective view of a second embodiment of the invention,
- Figure 5: is a perspective view of a third embodiment of the invention,
- Figure 6: is a perspective view of an absorbent article in the form of a diaper which is designed according to a fourth embodiment of the invention,
- Figure 7: is a cross-sectional view of the invention which in principle corresponds to the cross section according to Figure 2, but showing a further embodiment of the invention, and
- Figure 8: shows a perspective view in which the article has been formed in accordance with a further alternative embodiment.

### PREFERRED EMBODIMENTS

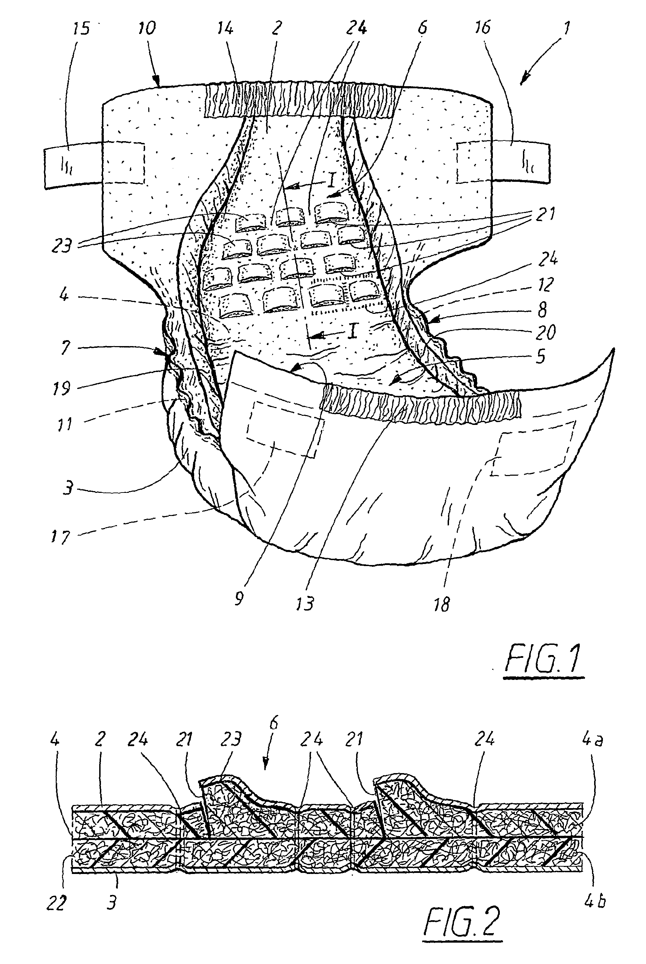

The present invention will now be described with reference to a number of preferred embodiments which indicate different types of absorbent articles in accordance with the invention. According to a first embodiment, which can be seen from Figure 1, the absorbent article is a diaper 1 of the disposable type for infants. The diaper 1 comprises a first cover sheet constituting a liquid-permeable top sheet 2. This top sheet 2 is arranged on that side of the diaper 1 which, during use, is intended to be directed towards the person wearing the diaper. The diaper 1 further comprises a second cover sheet constituting a bottom sheet 3 which, during use, is intended to be directed away from the user. The bottom sheet 3 is expediently liquid-tight. A layer of a wadding material 4 is arranged under the top sheet 2. In accordance with what is described in detail below, the top sheet 2 and the underlying wadding material 4 form a liquid-permeable and liquid-transporting laminate which, according to the invention, is used to take up loose and runny excrement in particular. Under the wadding material 4 there is an absorption body, which is not indicated by any reference number in Figure 1 but will be described below with reference in particular to Figure 2.

Thus, a structure is formed in the diaper according to the invention which, from the top downwards, consists of a cover sheet 2 and a wadding material 4 (which together constitute a laminate), an absorption body, and a bottom sheet 3.

The liquid-permeable top sheet 2 according to Figure 1 expediently consists of a fibrous material, for example a soft nonwoven material, but it can alternatively consist of other materials or material laminates. For example, it can consist of a perforated plastic film, for example of a thermoplastic material such as polyethylene or polypropylene, or a net-like layer of synthetic or textile material. Likewise, different types of laminates of suitable materials can be used as the liquid-permeable top sheet. The nonwoven materials used are preferably synthetic fibres such as polyethylene, polypropylene, polyester, nylon or the like. In addition, mixtures of different fibre types can be used for said nonwoven materials. The top sheet 2 expediently has a pore size which is large enough to let urine pass through even though the fibres in this sheet are hydrophobic.

Thus, the top sheet 2 is preferably a nonwoven material, but, irrespective of the material chosen, the top sheet 2 is intended in a manner known per se to receive and let through liquid excretions from the user and transfer these downwards to the underlying wadding material 4. The wadding material 4 is preferably a suitable hydrophilic material of specific resilience and thickness. Moreover, the underlying bottom sheet 3 is expediently liquid-impermeable and is intended to prevent any leakage of liquid through the wadding material 4 and the absorption body. For this purpose, the bottom sheet 3 can be made of a liquid-impermeable material expediently in the form of a thin and liquid-tight plastic film. For example, plastic films made of polyethylene, polypropylene or polyester can be used. Alternatively, a laminate of nonwoven and plastic film or other suitable material layers can be used as the liquid-tight bottom sheet 3.

As can be seen from Figure 1, the diaper 1 has a substantially elongate shape and is generally formed to fit around the lower trunk of an infant when in use. The diaper 1 can thus be said to be designed so that it defines a longitudinal direction and a transverse direction. The diaper 1 is designed with a front portion 5 in its longitudinal direction, and a rear portion 6 in its longitudinal direction. When the diaper 1 is in use, the front portion 5 is positioned so that it is directed towards the user's belly and down towards the groin area, while the rear portion 6 is positioned so that it is directed towards the user's buttocks. The boundary between the front portion 5 and rear portion 6 does not need to be defined with exact dimensions and does not occur at a specified transverse position for example, but instead along an extended transition area at the user's crotch region. As regards the function of the diaper 1, the rear portion 6 is basically designed to take up a greater amount of excrement from the user than is the front portion 5. The rear portion 6 can therefore be said to have a position and extent corresponding to the area where excrement may be expected to accumulate.

The diaper 1 is further designed with two elongate side edges 7, 8 and two transverse end edges 9, 10, more specifically a front end edge 9 and a rear end edge 10. The two side edges 7, 8 are designed with elastic arrangements 11, 12 (which are shown by broken lines in Figure 1), for example in the form of longitudinal elastic threads, bands or the like. In this way, elastic sections are defined, so-called leg elastics, at the side edges 7, 8, so that each side edge 7, 8 bears on and provides a seal against the inner surface of the user's leg in order to prevent leakage of urine and excrement.

Moreover, the diaper 1 comprises further elastic elements 13, 14, preferably in the form of two elasticated portions along the front end edge 9 and the rear end edge 10, respectively. These elastic elements 13, 14 are designed to close against the user's belly and back, respectively, as a result of which the diaper 1 is given the required fit and comfort during use.

To fit the diaper 1 on the user, it is additionally designed with two tape-like fastening strips 15, 16 which are arranged along respective side edges 7, 8 near the rear edge 10. The fastening strips 15, 16 are intended to cooperate and are secured in a releasable manner against corresponding fastening areas 17, 18 (which are indicated by broken lines in Figure 1) at the front end edge 9. The fastening strips 15, 16 can be secured on the fastening areas 17, 18 by means of the fastening strips 15, 16 being provided with a suitable adhesive for example, or alternatively by their being designed as velcro-type fasteners or in some other suitable manner.

The diaper is further designed with two longitudinal side barriers 19, 20 which run inside of each side edge 7, 8 and are designed as longitudinal edges extending up from the surface of the diaper 1, i.e. so that they prevent leakage of excrement and urine sideways out of the diaper 1. The side barriers 19, 20 are expediently provided with elastic elements (not shown in Figure 1) which are intended to raise the respective side barrier 19, 20 in a direction substantially at right angles from the plane of the top sheet 2. In this way, effective barriers are formed which prevent said leakage.

The diaper 1 according to the invention is especially suitable for newborn babies. In such an application, the length of the diaper 1 is less than 400 mm, while its width is less than 300 mm. However, the invention is not limited to any specific sizes as regards the external dimensions of the diaper, and instead it can be adapted to different users of different sizes.

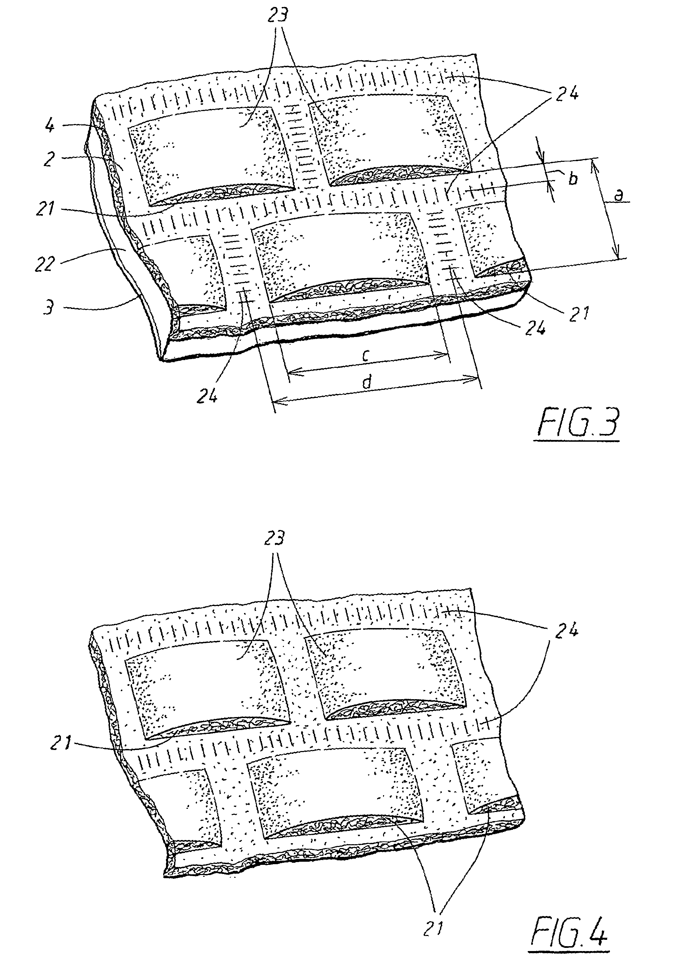

The basic principle behind the present invention will now be described in detail with reference to Figure 1 and Figure 2, where Figure 2 is a cross-sectional view along the line 1-1 in Figure 1. As is shown in the figures, the laminate consisting of the liquid-permeable top sheet 2 and the wadding material 4 is cut through at certain positions by a number of slits 21. Through these openings, the wadding material 4 is exposed for passage and accumulation of bodily excretions from the user. It should be noted that the openings which are defined by the slits 21 extend in the z direction of the diaper 1 (see in particular Figure 2), i.e. extend in a direction which is substantially at right angles to the plane along which the top sheet 2 is oriented. According to the embodiment shown, the slits 21 are placed in a uniform pattern, as can be seen from Figure 1, by which means the slits 21 and the formation of the top sheet 2 form a grid-like structure, which is preferably positioned at the rear portion 6 of the diaper 1. In this way, the openings which are defined by the slits 21 are positioned within that area of the diaper 1 where excrement from the user can be expected to accumulate during use of the diaper 1. According to the embodiment shown, the slits 21 are placed in a number of rows which all run along the transverse direction of the diaper 1. As will be described below with reference to Figures 3-7, a number of different configurations can be chosen for the position and design of these slits 21 within the scope of the invention.

As has been mentioned above, and as will be seen in particular from Figure 2, an absorption body 22 is arranged between the laminate and the bottom sheet 3, i.e. on top of the bottom sheet 3 but under the wadding material 4. In the embodiment according to Figure 2, the laminate, as has been mentioned above, consists of a top sheet 2 and a wadding material 4. According to an alternative embodiment, which is not shown in any of the figures, the laminate can further comprise a fibrous sheet under the wadding material. This further sheet is then also expediently a nonwoven (like the top sheet 2) and will be placed on top of the absorption body 22.

The main purpose of the position and design of the slits 21 is to allow excrement, which in the case of infants can be expected to be loose and runny in consistency, to pass quickly and efficiently through each slit 21 and down to the underlying wadding material 4. In this way, excrement can be taken up and bound to the wadding material 4. A further effect of the slits 21 is that they have a decelerating effect which to a great extent prevents excrement from leaking out at the user's back, i.e. past the rear end edge 10. By virtue of the top sheet 2 being provided with slits, the underlying wadding material 4 is exposed in the z direction in each opening formed. This opening at each hump thus slows down the spread of excrement and additionally defines a storage space for excrement, so that contact of the excrement with the user's skin is reduced.

By means of the position and design of the slits 21, and by virtue of the inherent resilience and thickness of the wadding material 4, mounds are formed, i.e. hump-shaped areas 23, in the top sheet 2, i.e. uniformly placed elevations of the material on the top face of the diaper 1. The height and elasticity of each hump-shaped area 23 is determined, inter alia, by the properties of the underlying wadding material 4.

By virtue of the slits 21 being positioned in the rear portion 6 of the diaper 1, the humps 23 are also positioned substantially under the user's buttocks. By virtue of the fact that the humps 23 have a certain extent in a direction out from the surface of the diaper 1, in the z direction, excrement is accumulated in the "valleys" which are defined between the humps 22. In addition, the wadding material 4 is less compressed in that part of the hump which adjoins the slit 21, for which reason excrement can be easily taken up by the wadding material. This means in turn that the user will feel the top sheet 2 of the diaper as very dry, which is of course an advantage as regards user comfort. In addition, this reduces the risk of the user experiencing skin irritation.

In the manufacture of the diaper, the abovementioned laminate is first obtained by means of the top sheet 2 being bound to the wadding material 4. This binding is preferably of the thermal binding kind, which can be used to create binding areas 24 (see Figure 2) which are then used to hold the top sheet 2 together with the wadding material 4. Thereafter, slits are formed by, for example, scissors, rotating blades, punching or the like, through substantially the whole laminate, whereupon the laminate is placed on the absorption body 22. In this way, the slits 21 are formed in the top sheet 2, and the underlying wadding material 4 is deformed by the slitting as is shown in Figure 2. The humps 23 are then formed as a consequence of the resilience or "spring" force of the wadding material 4. During slitting, the uppermost fibres in the wadding material 4 are cut so that this material springs back and rises in the opening which is formed. The positioning of the binding areas 24 in relation to the slits 21 is therefore of importance and is selected so as to obtain good resilience. It is therefore the resilience of the wadding material that gives the humps their height in the z direction.

The actual process of binding the laminate together and obtaining the slits in the laminate is not shown in detail here, but it can expediently be based on the laminate being obtained in the form of a material web which is first fed through a binding station for thermal binding and then through a slitting station where the laminate is cut through at suitable positions with a number of blades. After the binding of the cover sheet 2 to the wadding material 4, and after the laminate thus formed has been slit, the whole laminate is placed on the absorption sheet 22. Thereafter, the bottom sheet 3 can be applied. It is also possible to apply a further material sheet on the underside of the laminate, that is to say between the wadding material 4 and the absorption sheet 22. In this way, the openings in the laminate are prevented from spreading out in an undesired way in the x-y plane, which can happen in the event of inadvertent stretching of the material sheets, for example when applying the laminate on the absorption sheet 22.

The structure and function of the invention will now be explained in more detail with reference to Figure 2 and also Figure 3, which is a slightly enlarged perspective view of a section of the rear portion 6 of the diaper 1. Figures 2 and 3 show how the top sheet 2 is cut along certain areas so that it defines a number of slits 21, which provide an open passage extending in the z direction through the upper surface of the top sheet 2 and down to the underlying wadding material 4. The wadding material 4 expediently consists of a soft and relatively thick material, which functions as a transporting and distributing layer for liquid excrement and urine passing through the top sheet 2 (i.e. either through the slits 21 or through the top sheet 2 itself). The wadding material expediently consists of a continuous layer lying along the entire surface of the diaper 1. Alternatively, the wadding material can be placed at only part of, or parts of, the surface of the diaper. The absorption body 22 consists of a highly absorbent material, for optimal absorption and storage of bodily excretions.

According to the embodiment, the diaper 1 is designed so that the wadding material in the rear portion 6 of the diaper 1 is thicker than the wadding material in the front portion 5 (see Figure 1) of the diaper 1. More precisely, the wadding material has a much greater bulk (i.e. volume per unit of weight) in the rear portion 6 of the diaper 1 where most excrement is expected to accumulate and where the slits 21 in the top sheet 2 are also cut out. In this way, the wadding material layer 4 is used optimally to take up excrement, which of course can be expected to take place mainly in the rear portion 6.

For the wadding material layer 4 to be able to take up large amounts of liquid quickly, it has a fibre structure with relatively large pores, i.e. with a capillary structure which permits effective uptake of liquid. The wadding material layer 4 is preferably of the fibre wadding type which can be bound or unbound and which can be based on synthetic or natural fibres. Through suitable choice of wadding material, a desired structure of the humps 23 is also obtained. More specifically, the choice of fibres in the wadding material layer 4, and the latter's density and elasticity or resilience, determines the tendency of the wadding material 4 to press upwards in the areas around the slits 21, so that the abovementioned upwardly projecting humps 23 can be formed. Elasticity or resilience is to be understood as meaning that the material has an ability to assume, or seek to assume, its original thickness or height after compression. To obtain humps 23 which are high in relation to the level of the rest of the top sheet 2, and thus also obtain distinct and large openings defined by the slits 21, material with particularly high resilience can be used in the wadding material 4 at the rear portion 6 of the diaper 1. This will be described in detail below.

The wadding material layer 4 is primarily intended as a transport layer for rapid transport of bodily fluids down to the absorption body 22. In other words, the wadding material layer 4 does not have to be highly absorbent. The underlying high-absorbency layer 22 is, by contrast, intended for a high degree of absorption of liquid and, for this purpose, is expediently made of cellulose fluff pulp. The highly absorbent material in the absorption body 22 can also include so-called superabsorbents, which are polymer compounds with the ability to absorb several times their own weight of liquids, such as bodily fluids. The absorption body 22 can alternatively consist of absorbent synthetic fibres, or mixtures of natural fibres and synthetic fibres.

The absorption body 22 according to the invention can be designed as is shown in Figure 2, or it can alternatively be designed in a way in which superabsorbents are admixed. The invention can thus be realized both with an absorption body which is defined by different sheets of material or a single sheet with good properties as regards the uptake, spread and storage of bodily fluids.

As is shown in Figures 2 and 3, the diaper 1 is preferably joined together by means of ultrasound welding so that binding areas 24 are defined. These binding areas 24 consist of a number of lines which bind the top sheet 2 to the wadding material 4 and are placed in a predefined manner close to the slits 22. These binding areas 24 are indicated also in Figure 1. As can also be seen in Figure 2 and Figure 3, these binding areas 24 are positioned so that they hold the material layers 2, 4 together about the humps 23 formed on the top face of the diaper 1. As can be seen in particular from Figure 3, these binding areas 24 additionally define a kind of grid system with straight lines in the longitudinal direction and transverse direction of the diaper 1. These lines surround the various humps 23.

As has been stated above, the diaper 1 is joined together expediently by thermal binding. However, the invention is not limited to this method, and instead the joining can also be done by means of adhesive binding or some other suitable method of forming the above-described binding areas 24. Regardless of which joining method is chosen, each binding area 24 can be designed as a series of discrete points, continuous lines, wide fields or other similar configurations. Various parameters such as the width of the binding areas (in the case where these are wide fields) or the position and dimensions of the points (in the case of a series of points) can also be varied within the scope of the invention. To obtain a strong bond between the material sheets, it is possible to choose the fibre materials in the cover sheet 2 and the wadding material 4 in such a way that fibres from both sheets are melted together at the binding areas 24.

As regards the width of each binding area 24, it can be stated that this is expediently of the order of 0.5-15 mm in a suitable design of a diaper according to the invention. The binding areas 24 are also positioned in a predetermined manner so that the slits 21 (and thus also the humps 23) will be able to be formed in accordance with the invention. As can be seen from Figure 3, two specific dimensions, a and b, can be defined in the longitudinal direction of the diaper. It is assumed here that the binding areas 24 are designed as a grid system with substantially straight lines extending in the longitudinal direction and transverse direction of the diaper. It is additionally assumed that the slits 21 are placed in line with one another in the transverse direction of the diaper 1. The first-mentioned dimension a relates to the distance between two neighbouring slits 21, as seen in the longitudinal direction of the diaper. The last-mentioned dimension b relates to the distance between a slit 21 and the binding area 24 positioned in front of the slit 21. According to the embodiment shown in Figures 1-3, the distance a is of the order of 10-100 mm, preferably 15-50 mm, while the distance b is of the order of up to 10 mm, preferably 1-10 mm.

As is shown in Figure 3, each slit 21 additionally defines a length c, that is to say in the transverse direction of the diaper, which is preferably of the order of 10-20 mm. This means that the binding areas 24 which run in substantially the longitudinal direction of the diaper between two neighbouring humps 23 are arranged at a distance d which slightly exceeds the length c of each slit 21.

The details of the various preferred dimensions a, b, c, d given here can also be applied to the embodiments which will be described below with reference to Figures 4-7.

According to a further embodiment of the invention, which is shown in Figure 4, there are no binding areas running in the longitudinal direction of the diaper 1, and instead the only binding areas 24 which are used run in the transverse direction of the diaper. The positioning and length of the slits 21 provide the conditions for forming humps of the type which have been described above.

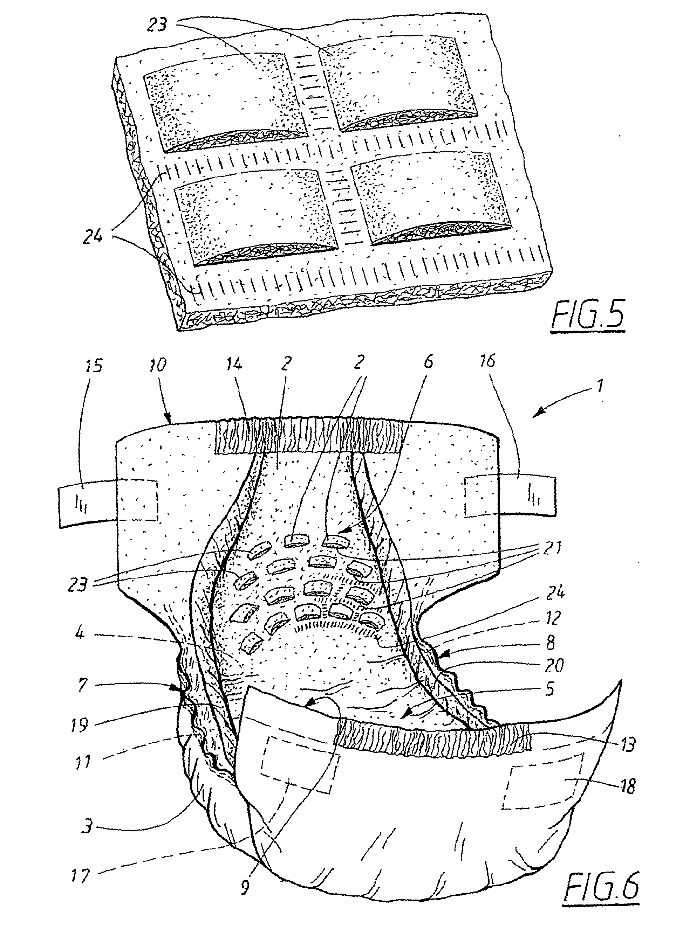

According to a third embodiment of the invention, which is shown in Figure 5, not every second row of humps 23 is offset in the transverse direction of the diaper, as is the case in the embodiments according to Figures 3 and 4, and instead the humps 23 are placed as regular grids across the surface of the diaper 1. In other words, the humps 23 lie in line with one another both in the transverse direction and in the longitudinal direction of the diaper. This third embodiment is provided with binding areas 24 in the longitudinal direction and the transverse direction of the diaper, or, alternatively, only in the transverse direction of the diaper (see Figure 3).

According to a fourth embodiment of the invention, which is shown in a perspective view in Figure 6, the humps 23 can be placed in an arrangement which, in contrast to Figures 3-5, is not oriented along straight lines. Instead, the humps 23 here are placed along an arc-like or fan-like structure in order in this way to adapt the ability of the diaper 1 to accumulate excrement to the body shape of the person using the diaper 1. In this embodiment, the openings which are defined by the slits 21 are thus directed more distinctly inwards towards the crotch region of the user.

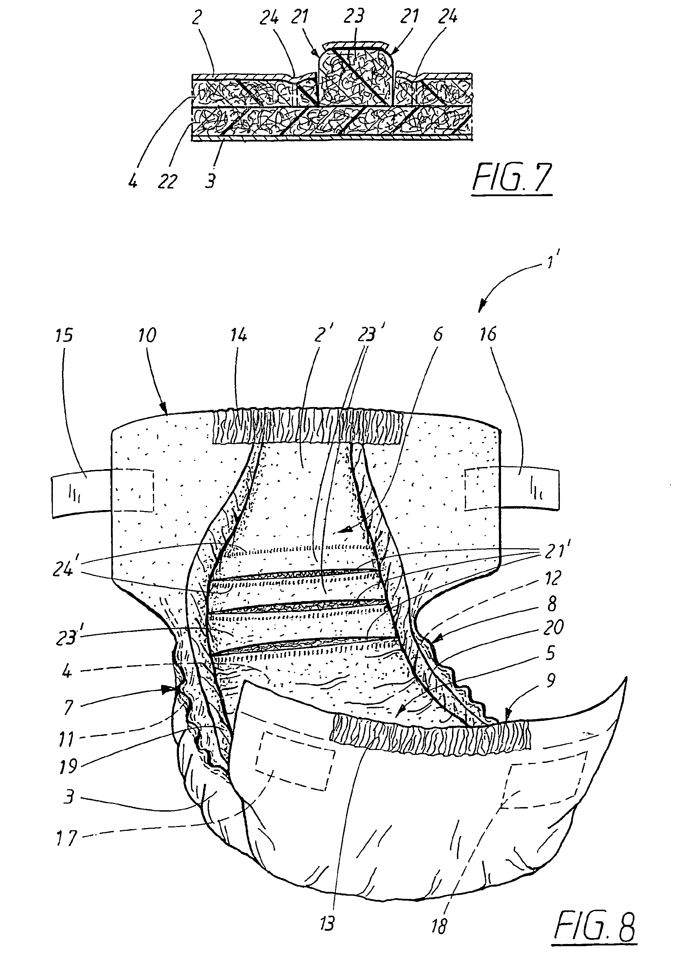

Figure 7 is a cross-sectional view which essentially corresponds to Figure 2, but which shows a further embodiment of the invention in which two slits 21 are made at each hump 23. Here, therefore, the material is slit at both the front edge and the rear edge of each hump 23, instead of only at the front edge, which is the case with the abovementioned embodiments (see Figure 2 for example). In this embodiment, the top sheet 2 defines a kind of arc-like shape on each hump 23. The alternative embodiments which are shown in Figures 4-6 can also be designed with double slits as shown in Figure 7.

In the embodiment which is shown in Figure 7, the distance between two slits 21 (i.e. across the hump 23) can be of the order of 4-100 mm, preferably 10-40 mm. The distance between two slits, viewed across the binding area 24, can moreover be of the order of 3-20 mm, preferably 5-15 mm.

It will be noted that although the binding areas 24 can consist of the straight lines shown in Figures 3-5, other configurations of weld seams can alternatively be chosen, for example short "broken" lines or points, which are then positioned in such a way as to permit suitable formation of the humps 23. It will likewise be noted that the weld seams do not have to be in the form of straight lines, and instead curved weld seams may be expedient in some applications (see Figure 6). The overall requirement satisfied by the invention is that of ensuring that the slits 21 define openings which extend in the z direction of the diaper 1 and permit optimal passage, to the wadding material 4, of the excrement collecting in the rear portion 6 of the diaper 1. It should be noted here that the openings defined at the slits 21 are so configured that an especially effective uptake of excrement is obtained in a direction along the diaper 1 running rearwards and substantially parallel to the top sheet 2 (see in particular Figure 2). In this way, excrement is transferred substantially straight in to the openings at the slits 21.

According to the embodiment shown, the humps 23 are placed in the form of substantially rectangular grids which are ordered in a number of substantially parallel rows along the longitudinal direction of the diaper. These rows extend transversely across the diaper 1, i.e. between the two side barriers 19, 20 of the diaper (see Figure 1). Every second row of humps 23 is preferably slightly offset, in the transverse direction of the diaper, in relation to the neighbouring row(s). In this way, the humps 23 are positioned so that they form a staggered and "overlapping" pattern so that a hump 23 in a given row lies in line with a gap between two humps 23 of a neighbouring row. This means in turn that the slits 21 can provide maximum access to loose excrement which is then allowed to pass to the underlying wadding material 4.

The position and dimensions of the slits 21 and humps 23 can vary within the scope of the invention. As an alternative to the relatively uniform patterns shown in Figures 1-7, the humps 23 can form any desired configurations which provide the desired uptake of excrement in particular. Likewise, the dimensions of the slits 21 and humps 23 can vary within the scope of the invention. Nor, for example, do the humps have to be rectangular or square, and instead they can be another shape, for example semicircular. Moreover, the humps do not all have to be the same size and shape, and instead different types of humps can be present in different areas of the rear portion 6 of the diaper.

The height of the humps 23 above the surface of the top sheet 2 can also vary within the scope of the invention. A greater height generally gives better access to the underlying wadding material 4, which, as is shown for example in Figure 3, can be discerned in the openings which are defined by the slits 21. A greater height of the humps 23 also gives the user an improved feeling of dryness, because excrement is then accumulated to a greater extent in the valleys between the humps 23, which in turn slows down the spread of the excrement and additionally isolates the user's skin from contact with the excrement.

An additionally improved effect can be achieved if the top sheet 2 is slit in at least part of the transverse gable ends of each hump 23, i.e. so that a greater part of the "grid" surrounding each hump 23 is opened for exposure of the underlying wadding material 4. The wadding material 4 will then also lift the top sheet 2 to a greater extent, so that the humps 23 are effectively raised. In addition, gables are thus formed where it is open directly into the wadding material 4.

According to an alternative embodiment, humps can be formed by punching squares from a complete sheet of wadding material. The remaining wadding material then defines humps. These are covered by cover material which, for example, is welded or glued firmly in the valleys between the humps and is slit where the wadding squares lie.

A principle behind the invention is that the inherent elasticity and hardness of the wadding material 4, and thus also its resilient properties, create conditions for forming the abovementioned humps 23. A further factor determining the form of the humps 23 is the thickness of the wadding material 4, and also the positioning and dimensioning of the slits and the binding areas.

Practical trials have shown that a permanent compression of less than 20% and a basis weight of 60-200 g/m² can be suitable material parameters for wadding material used in connection with the invention. A measure of the permanent compression is obtained by first measuring the difference between the thickness of the material after a certain compression force has been applied and thereafter removed (the material having been allowed to recover) and the original thickness of the material. This value is then divided by the original thickness and gives a value of the permanent compression of the material in question. A customary measurement method for permanent compression is ISO 1856:1980.

An alternative embodiment of the invention will now be described with reference to Figure 8 which shows a diaper 1' of substantially the same type as is shown in Figure 1, but where the top sheet 2' of the diaper is designed in an alternative way. The components in Figure 8 which correspond to like components in Figure 1 have been indicated by the same reference numbers as in Figure 1. In contrast to the embodiments shown in Figures 1-7, Figure 8 describes a variant of the invention in which relatively long slits 21' have been formed and run transversely with respect to the longitudinal direction of the diaper 1'. More precisely, in this embodiment a number of these transverse slits 21' have been used which preferably run between respective side barriers 19, 20 of the diaper 1'. In Figure 8, three such slits 21' are shown, but the number of slits 21' can vary within the scope of the invention.

In the embodiment according to Figure 8, the different material layers of the diaper 1' are also joined together at suitable positions. Ultrasound welding is expediently used to form a number of binding areas 24', expediently in the form of a long line running in front of each slit 21'. In this way, a joined structure is formed with large transverse openings in the z direction, which facilitate uptake of excrement in an underlying wadding material 4.

The end portion of each slit 21' can be secured in the respective side barrier 19, 20 of the diaper 1'. In this way, each end portion of the slits 21' will lift together with the side barriers 19, 20 in an effective manner. As in the case of the hump-shaped areas 23 shown in Figures 1-3, the height of the transverse raised areas 23' according to Figure 8 is also affected by the properties of the materials included in the underlying wadding material 4, e.g. its bulk, elasticity and resilience.

Figure 8 shows how, at the openings which are defined at each slit 21', the material in the underlying wadding material 4 is exposed. As in the embodiments according to Figures 1-7, the main principle of the function of the diaper 1' according to Figure 8 is that the top sheet 2' is provided with slits 21' of such a type that the wadding material 4 is exposed for passage of bodily excretions through said slits 21'. The embodiment according to Figure 4 is especially effective as regards the ability to slow down excrement so that the latter does not leak out via the rear end edge 10 of the diaper 1'.

As regards dimensions, the embodiment according to Figure 8 is designed so that the length of the slits 21' is preferably substantially the same as or slightly smaller than the width of the finished diaper 1' between the side barriers 19, 20. In addition, these long transverse slits can alternatively be curved, i.e. as an alternative to the substantially straight slits 21' shown in Figure 8.

The invention is not limited to the embodiments described above and instead it can be varied within the scope of the attached claims. For example, the principle behind the invention can be applied not just to diapers, but also, for example, to incontinence shields for collecting urine and excrement from adults suffering from incontinence. Likewise, the invention can in principle be used in sanitary towels.

The components involved can have different dimensions. For example, the size, height and position of the humps (according to Figures 1-7) and the raised areas (according to Figure 8) can vary. Likewise, the wadding material 4 can be designed in different ways, for example with different types of material of suitably chosen dimensions and material properties.

The structures which have been described above can also be combined with other suitable constructions for collecting bodily excretions. For example, an absorbent article according to the invention can be supplemented with other types of pockets, barrier structures and surface material in order to further improve the function as regards collection of excrement, for example.

## Claims

1. Absorbent article (1; 1') such as a diaper, an incontinence shield, a sanitary towel or the like, comprising an upper, liquid-permeable cover sheet (2), a lower, preferably liquid-impermeable cover sheet (3), and an absorption body (22) arranged between the cover sheets (2, 3), said article (1; 1') defining a longitudinal direction, a front portion (5) in the longitudinal direction, and a rear portion (6) in the longitudinal direction, **characterized in that** the upper cover sheet (2), together with an underlying wadding material (4), defines a laminate which, at least in said rear portion (6), is designed with at least one through-slit (21; 21') being formed by cutting through said top sheet (2) and said wadding material (4), thereby defining a corresponding opening for passage of bodily excretions through said slit (21; 21') and said wadding material (4), and **in that** said slit (21; 21') is surrounded by binding areas (24) which, together with a resilient action of the wadding material (4), define a raised area (23; 23') on the surface of said article (1; 1').

2. Absorbent article (1; 1') according to claim 1, **characterized in that** the opening which is defined at said slit (21; 21') extends in a direction which is substantially at right angles to a plane along which the surface of the article (1; 1') extends.

3. Absorbent article (1; 1') according to either of the preceding claims, **characterized in that** the opening which is defined at each slit (21; 21') faces substantially in the direction towards said front portion (5) of the article (1).

4. Absorbent article (1; 1') according to any of the preceding claims, **characterized in that** said binding areas (24) are formed by thermal binding.

5. Absorbent article (1; 1') according to any of the preceding claims, **characterized in that** said binding areas (24) define lines, points or elongate areas extending along a number of substantially parallel lines in the transverse direction of said article (1; 1').

6. Absorbent article (1; 1') according to any of the preceding claims, **characterized in that** said binding areas (24) define lines, points or elongate areas extending along a number of substantially parallel lines in the longitudinal direction of said article (1; 1').

7. Absorbent article (1; 1') according to any of claims 1-4, **characterized in that** said binding areas (24) define lines, points or elongate areas which define an arc-shaped configuration so that the openings which are defined by each slit (21; 21') face towards an imagined position for the crotch region of the person using said article (1; 1').

8. Absorbent article (1; 1') according to any of claims 5-7, **characterized in that** it comprises a slit (21; 21') between two binding areas (24).

9. Absorbent article (1; 1') according to any of claims 5-7, **characterized in that** it comprises two slits between two binding areas (24).

10. Absorbent article (1) according to any of the preceding claims, **characterized in that** it comprises a number of slits (21) which are oriented so that a corresponding number of raised areas (23) define humps which are formed along a number of rows running substantially at right angles to said longitudinal direction of the article (1), neighbouring rows being offset so that a hump (23) in one row is in line with a gap between two humps (23) of a neighbouring row.

11. Absorbent article (1; 1') according to any of the preceding claims, **characterized in that** each slit (1; 1') is positioned such that said wadding material (4) is exposed for taking up bodily excretions with a direction of spread which is substantially parallel to said upper cover sheet (2; 2').

12. Absorbent article (1; 1') according to any of the preceding claims, **characterized in that** said slit (1; 1') is positioned substantially at said rear portion (6) of said article (1; 1'), said rear portion (6) being defined as an area at which excrement may be expected to accumulate.

13. Absorbent article (1; 1') according to any of the preceding claims, **characterized in that** said wadding material (4) has a substantially greater bulk in said rear portion (6) than in the rest of the article.

14. Absorbent article (1; 1') according to any of the preceding claims, **characterized in that** the distance between two neighbouring binding areas in the longitudinal direction of the article is of the order of 10-100 mm, preferably 15-50 mm.

15. Absorbent article (1; 1') according to any of the preceding claims, **characterized in that** the distance between a binding area (24) and a slit (21) behind it in the longitudinal direction of the article is of the order of up to 10 mm, preferably 1-10 mm.

16. Absorbent article (1; 1') according to any of the preceding claims, **characterized in that** the length of said slit (21) in the transverse direction of the article is of the order of 10-20 mm.

17. Method for manufacturing an absorbent article (1; 1') such as a diaper, an incontinence shield, a sanitary towel or the like, comprising an upper, liquid-permeable cover sheet (2), a lower, liquid-impermeable cover sheet (3), and an absorption body (22) arranged between the cover sheets (2, 3), said article (1; 1') defining a longitudinal direction, a front portion (5) in the longitudinal direction, and a rear portion (6) in the longitudinal direction, **characterized in that** the method comprises:
joining together a laminate which consists of said upper cover sheet (2) in conjunction with an underlying wadding material (4),
forming binding areas (24) which will surround said slit (21; 21') and which, together with a resilient action of the wadding material (4), define raised areas (23; 23') on the surface of said article (1; 1'), and
forming at least one slit (21; 21') in said laminate over a predetermined surface (6) along said article, by cutting through said top sheet (2) and said wadding material (4), by which means a corresponding opening is defined for passage of bodily excretions through said slit (21; 21') and said wadding material (4).

18. Method for manufacturing an absorbent article (1; 1') in accordance with claim 17, **characterized in that** said formation of binding areas (24) comprises a process of thermal binding.

19. Method for manufacturing an absorbent article (1; 1') in accordance with claim 18, **characterized in that** fibres from both the cover sheet (2) and the wadding material (4) are melted together at the binding areas (24).

## Patentansprüche

1. Absorbierender Gegenstand (1; 1'), wie beispielsweise eine Windel, ein Inkontinenzschutz, eine Damenbinde oder ähnliches, umfassend eine obere flüssigkeitsdurchlässige Decklage (2), eine untere vorzugsweise flüssigkeitsundurchlässige Decklage (3) und einen Absorptionskörper (22), der zwischen den Decklagen (2, 3) angeordnet ist, wobei der Gegenstand (1; 1') eine Längsrichtung, einen in Längsrichtung vorderen Abschnitt (5) und einen in Längsrichtung hinteren Abschnitt (6) definiert, **dadurch gekennzeichnet, dass** die obere Decklage (2) zusammen mit einem darunter liegenden Füllmaterial (4) einen Verbund definiert, der wenigstens in dem besagten hinteren Abschnitt (6) mit wenigstens einem durchgehenden Schlitz (21, 21') ausgestaltet ist, der durch Durchtrennen der Oberlage (2) und dem Füllstoffmaterial (4) ausgebildet ist, wodurch eine entsprechende Öffnung zum Durchgang von Körperausscheidungen durch den besagten Schlitz (21, 21') und das besagte Füllmaterial (4) definiert wird und **dadurch**, dass der besagte Schlitz (21, 21') von bindenden Bereichen (24) umgeben ist, die zusammen mit einer elastischen Wirkung des Füllstoffmaterials (4) einen aus der Oberfläche des Gegenstandes (1, 1') erhabenen Bereich (23, 23') definieren.

2. Absorbierender Gegenstand (1, 1') nach Anspruch 1, **dadurch gekennzeichnet, dass** sich die Öffnung, die an dem besagten Schlitz (21, 21') definiert ist in einer Richtung erstreckt, die im Wesentlichen im rechten Winkel zu einer Ebene verläuft, entlang der sich die Oberfläche des Gegenstandes (1, 1') erstreckt.

3. Absorbierender Gegenstand (1, 1') nach einem der vorstehenden Ansprüche, **dadurch gekennzeichnet, dass** die Öffnung, die an jedem Schlitz (21, 21') definiert ist im Wesentlich in einer Richtung zum vorderen Abschnitt (5) des Gegenstandes (1) weist.

4. Absorbierender Gegenstand (1, 1') nach einem der vorstehenden Ansprüche, **dadurch gekennzeichnet, dass** die bindenden Bereiche (24) durch thermisches Binden ausgebildet sind.

5. Absorbierender Gegenstand (1, 1') nach einem der vorstehenden Ansprüche, **dadurch gekennzeichnet, dass** die bindenden Bereiche (24) Linienpunkte oder längliche Bereiche, die sich entlang einer Anzahl im Wesentlichen paralleler Linien in Querrichtung des Gegenstandes (1, 1') erstrecken, definieren.

6. Absorbierender Gegenstand (1, 1') nach einem der vorstehenden Ansprüche, **dadurch gekennzeichnet, dass** die bindenden Bereiche (24) Linienpunkte oder längliche Bereiche, die sich entlang einer Anzahl im Wesentlichen paralleler Linien in Längsrichtung des Gegenstandes (1, 1') erstrecken, definiert.

7. Absorbierender Gegenstand (1, 1') nach einem der Ansprüche 1-4, **dadurch gekennzeichnet, dass** die bindenden Bereiche (24) Linienpunkte oder längliche Bereiche, die eine bogenförmige Konfiguration definieren, so dass die Öffnungen, die durch jeden Schlitz (21, 21') definiert sind in Richtung einer imaginären Position des Schrittbereichs der Person, die den Gegenstand (1, 1') verwendet, weisen.

8. Absorbierender Gegenstand (1, 1') nach einem der Ansprüche 5-7, **dadurch gekennzeichnet, dass** er einen Schlitz (21, 21') zwischen zwei bindenden Bereichen (24) umfasst.

9. Absorbierender Gegenstand (1, 1') nach einem der Ansprüche5-7, **dadurch gekennzeichnet, dass** er zwei Schlitz (21, 21') zwischen zwei bindenden Bereichen (24) umfasst.

10. Absorbierender Gegenstand (1, 1') nach einem der vorstehenden Ansprüche, **dadurch gekennzeichnet, dass** er eine Anzahl an Schlitzen (21) umfasst, die derart ausgerichtet sind, dass eine entsprechende Anzahl erhabener Bereiche (23) Höcker definiert, die entlang einer Anzahl von Reihen ausgebildet sind, die im Wesentlichen in rechten Winkeln zu der besagten Längsrichtung des Gegenstandes (1) verlaufen, wobei benachbarte Reihen versetzt sind, so dass ein Höcker (23) in einer Reihe mit einem Spalt zwischen zwei Höckern (23) einer benachbarten Reihe liegt.

11. Absorbierender Gegenstand (1, 1') nach einem der vorstehenden Ansprüche, **dadurch gekennzeichnet, dass** jeder Schlitz (1, 1') derart positioniert ist, dass das Füllstoffmaterial (4) freiliegt, um Körperausscheidungen mit einer Verteilungsrichtung, die im Wesentlichen parallel zu der besagten oberen Decklage (2, 2') verläuft, aufzunehmen.

12. Absorbierender Gegenstand (1, 1') nach einem der vorstehenden Ansprüche, **dadurch gekennzeichnet, dass** der Schlitz (1, 1') im Wesentlichen in dem hinteren Abschnitt (6) des Gegenstandes (1, 1') positioniert ist, wobei der hintere Abschnitt (6) als ein Bereich definiert ist, in dem es zu erwarten ist, dass sich Exkremente Ansammeln.

13. Absorbierender Gegenstand (1, 1') nach einem der vorstehenden Ansprüche, **dadurch gekennzeichnet, dass** das Füllstoffmaterial (4) ein wesentliches größeres Volumen in dem besagten hinteren Abschnitt (6) aufweist als im Rest des Gegenstandes.

14. Absorbierender Gegenstand (1, 1') nach einem der vorstehenden Ansprüche, **dadurch gekennzeichnet, dass** der Abstand zwischen zwei benachbarten bindenden Bereichen in Längsrichtung des Gegenstandes der Größenordnung von 10 bis 100 mm, vorzugsweise 15 bis 50 mm, liegt.

15. Absorbierender Gegenstand (1, 1') nach einem der vorstehenden Ansprüche, **dadurch gekennzeichnet, dass** der Abstand zwischen einem bindendem Bereich (24) und einem Schlitz (21) hinter ihm in Längsrichtung des Gegenstandes in der Größenordnung von bis zu 10 mm, vorzugsweise 1 bis 10 mm, liegt.

16. Absorbierender Gegenstand (1, 1') nach einem der vorstehenden Ansprüche, **dadurch gekennzeichnet, dass** die Länge des Schlitzes (21) in Querrichtung des Gegenstandes die der Größenordnung von 10 bis 20 mm liegt.

17. Verfahren zum Herstellen eines absorbierenden Gegenstandes (1, 1') wie beispielsweise einer Windel, einem Inkontinenzschutz, einer Damenbinde oder ähnlichem, umfassend eine obere flüssigkeitsdurchlässig Decklage (2), eine untere flüssigkeitsundurchlässige Decklage (3) und einen Absorptionskörper (22), der zwischen den Decklagen (2, 3) angeordnet ist, wobei der Gegenstand (1, 1') eine Längsrichtung, einen in Längsrichtung vorderen Abschnitt (5) und einen in Längsrichtung hinteren Abschnitt (6) definiert, **dadurch gekennzeichnet, dass** das Verfahren umfasst:
miteinander Verbinden eines Laminats, das aus der oberen Decklage (2) in Verbindung mit einem darunter liegenden Füllstoffmaterial (4) besteht,
Ausbilden von bindenden Bereichen (24), die die Schlitze (21, 21') umgeben werden und die zusammen mit einer elastischen Wirkung des Füllstoffmaterials (4) auf der Oberfläche des Gegenstandes (1, 1') erhabene Bereiche (2:3, 23') definieren, und
Bilden wenigstens eines Schlitzes (21, 21') in dem Verbund über einer vorbestimmten Fläche (6) entlang des Gegenstandes, in dem die Decklage (2) und das Füllstoffmaterial (4) durchtrennt werden, wodurch eine entsprechende Öffnung zum Durchgang von Körperausscheidungen durch den Schlitz (21, 21') und das Füllstoffmaterial (4) definiert wird.

18. Verfahren zum Herstellen eines absorbierenden Gegenstandes (1, 1') nach Anspruch 17, **dadurch gekennzeichnet, dass** die Ausbildung der bindenden Bereiche (24) einen thermischen Bindungsvorgang umfasst.

19. Verfahren zum Herstellen eines absorbierenden Gegenstandes (1, 1') nach Anspruch 18, **dadurch gekennzeichnet, dass** Fasern aus sowohl der Decklage (2) als auch dem Füllstoffmaterial (4) in den bindenden Bereichen (24) miteinander verschmolzen werden.

## Revendications

1. Article absorbant (1 ; 1'), tel qu'une couche-culotte, une protection contre l'incontinence, une serviette hygiénique ou article similaire, comprenant une feuille de couverture supérieure, perméable aux liquides, (2), une feuille de couverture inférieure, de préférence imperméable aux liquides, (3), et un corps absorbant (22) placé entre les feuilles de couverture (2, 3), ledit article (1 ; 1') définissant une direction longitudinale, une partie avant (5) dans la direction longitudinale et une partie arrière (6) dans la direction longitudinale, **caractérisé en ce que** la feuille de couverture supérieure (2), avec un matériau de rembourrage sous-jacent (4), définit une structure stratifiée qui, au moins dans ladite partie arrière (6), est pourvue d'au moins une fente traversante (21 ; 21') qui est formée en coupant ladite feuille supérieure (2) et ledit matériau de rembourrage (4), définissant ainsi une ouverture correspondante pour le passage d'excrétions corporelles par ladite fente (21 ; 21') et à travers ledit matériau de rembourrage (4), et **en ce que** ladite fente (21 ; 21') est entourée par des zones de liaison (24) qui, avec une action élastique du matériau de rembourrage (4), définissent une zone en relief (23 ; 23') à la surface dudit article (1 ; 1').

2. Article absorbant (1 ; 1') selon la revendication 1, **caractérisé en ce que** l'ouverture qui est définie au niveau de ladite fente (21 ; 21') s'étend dans une direction qui est substantiellement à angle droit par rapport à un plan dans lequel s'étend la surface de l'article (1 ; 1').

3. Article absorbant (1 ; 1') selon l'une quelconque des revendications précédentes, **caractérisé en ce que** l'ouverture qui est définie au niveau de chaque fente (21 ; 21') est tournée substantiellement en direction de ladite partie avant (5) de l'article (1).

4. Article absorbant (1 ; 1') selon l'une quelconque des revendications précédentes, **caractérisé en ce que** lesdites zones de liaison (24) sont formées par soudage thermique.

5. Article absorbant (1 ; 1') selon l'une quelconque des revendications précédentes, **caractérisé en ce que** lesdites zones de liaison (24) définissent des lignes, des points ou des zones allongées qui s'étendent le long d'un certain nombre de lignes sensiblement parallèles dans le sens transversal dudit article (1 ; 1').

6. Article absorbant (1 ; 1') selon l'une quelconque des revendications précédentes, **caractérisé en ce que** lesdites zones de liaison (24) définissent des lignes, des points ou des zones allongées qui s'étendent le long d'un certain nombre de lignes sensiblement parallèles dans le sens longitudinal dudit article (1 ; 1').

7. Article absorbant (1 ; 1') selon l'une quelconque des revendications 1 à 4, **caractérisé en ce que** lesdites zones de liaison (24) définissent des lignes, des points ou des zones allongées qui définissent une configuration en forme d'arc, de sorte que les ouvertures qui sont définies par chaque fente (21 ; 21') sont orientées vers une position imaginée pour la région d'entrejambe de la personne qui utilise ledit article (1 ; 1').

8. Article absorbant (1 ; 1') selon l'une quelconque des revendications 5 à 7, **caractérisé en ce qu'**il comprend une fente (21 ; 21') entre deux zones de liaison (24).

9. Article absorbant (1 ; 1') selon l'une quelconque des revendications 5 à 7, **caractérisé en ce qu'**il comprend deux fentes entre deux zones de liaison (24).

10. Article absorbant (1 ; 1') selon l'une quelconque des revendications précédentes, **caractérisé en ce qu'**il comprend un certain nombre de fentes (21) qui sont orientées de telle manière qu'un nombre correspondant de zones en relief (23) définissent des bosses qui sont formées le long d'un certain nombre de rangées sensiblement à angle droit par rapport à ladite direction longitudinale de l'article (1), les rangées voisines étant décalées de telle manière qu'une bosse (23) d'une rangée est alignée avec un espace entre deux bosses (23) d'une rangée voisine.

11. Article absorbant (1 ; 1') selon l'une quelconque des revendications précédentes, **caractérisé en ce que** chaque fente (1 ; 1') est positionnée de telle manière que ledit matériau de rembourrage (4) est exposé pour prendre les excrétions corporelles avec une direction de progression qui est sensiblement parallèle à ladite feuille de couverture supérieure (2 ; 2').

12. Article absorbant (1 ; 1') selon l'une quelconque des revendications précédentes, **caractérisé en ce que** ladite fente (1 ; 1') est positionnée substantiellement dans ladite partie arrière (6) dudit article (1 ; 1'), ladite partie arrière (6) étant définie comme une zone où l'on s'attend à ce que des excréments s'accumulent.

13. Article absorbant (1 ; 1') selon l'une quelconque des revendications précédentes, **caractérisé en ce que** ledit matériau de rembourrage (4) a un volume massique substantiellement plus grand dans ladite partie arrière (6) que dans le reste de l'article.

14. Article absorbant (1 ; 1') selon l'une quelconque des revendications précédentes, **caractérisé en ce que** la distance entre deux zones de liaison voisines dans le sens longitudinal de l'article est de l'ordre de 10 à 100 mm, de préférence de 15 à 50 mm.

15. Article absorbant (1 ; 1') selon l'une quelconque des revendications précédentes, **caractérisé en ce que** la distance entre une zone de liaison (24) et une fente (21) derrière elle dans le sens longitudinal de l'article est de l'ordre de 10 mm maximum, de préférence entre 1 et 10 mm.

16. Article absorbant (1 ; 1') selon l'une quelconque des revendications précédentes, **caractérisé en ce que** la longueur de ladite fente (21) dans le sens transversal de l'article est de l'ordre de 10 à 20 mm.

17. Procédé de fabrication d'un article absorbant (1 ; 1') tel qu'une couche-culotte, une protection contre l'incontinence, une serviette hygiénique ou article similaire, comprenant une feuille de couverture supérieure, perméable aux liquides, (2), une feuille de couverture inférieure, imperméable aux liquides, (3), et un corps absorbant (22) placé entre les feuilles de couverture (2, 3), ledit article (1 ; 1') définissant une direction longitudinale, une partie avant (5) dans la direction longitudinale et une partie arrière (6) dans la direction longitudinale, **caractérisé en ce que** le procédé comprend :
la formation par liaison d'une structure stratifiée qui est constituée de ladite feuille de couverture supérieure (2) associée à un matériau de rembourrage sous-jacent (4),
la formation de zones de liaison (24) qui vont entourer ladite fente (21 ; 21') et qui, avec une action élastique du matériau de rembourrage (4), définissent des zones en relief (23 ; 23') à la surface dudit article (1 ; 1'), et
la formation d'au moins une fente (21 ; 21') dans ladite structure stratifiée sur une surface prédéterminée (6) sur ledit article, en coupant ladite feuille supérieure (2) et ledit matériau de rembourrage (4), moyennant quoi une ouverture correspondante est définie pour le passage d'excrétions corporelles par ladite fente (21 ; 21') et à travers ledit matériau de rembourrage (4).

18. Procédé de fabrication d'un article absorbant (1 ; 1') selon la revendication 17, **caractérisé en ce que** ladite formation de zones de liaison (24) comprend un processus de soudage thermique.

19. Procédé de fabrication d'un article absorbant (1 ; 1') selon la revendication 18, **caractérisé en ce que** des fibres de la feuille de couverture (2) et du matériau de rembourrage (4) fusionnent au niveau des zones de liaison (24).
